(19) Europäisches Patentamt
European Patent Office
Office européen des brevets

(11) **EP 1 747 799 A1**

(12) **EUROPEAN PATENT APPLICATION**

(43) Date of publication:
**31.01.2007 Bulletin 2007/05**

(51) Int Cl.:
*A61N 5/10* (2006.01)   *G01T 1/29* (2006.01)

(21) Application number: **05447206.3**

(22) Date of filing: **19.09.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **27.07.2005 EP 05447177**

(71) Applicant: **ION BEAM APPLICATIONS S.A.**
**1348 Louvain-La-Neuve (BE)**

(72) Inventors:
• **Brusasco, Caterina**
  **5032 Bossiere (BE)**
• **Marchetto, Flavio**
  **10071 Borgaro Torinese (TO) (IT)**

(74) Representative: **Van Malderen, Joëlle et al**
  **pronovem - Office Van Malderen**
  **Avenue Josse Goffin 158**
  **1082 Bruxelles (BE)**

(54) **Dosimetry device for verification of a radiation therapy apparatus**

(57)    The present invention relates to a dosimetry device having a configuration allowing accelerator factory testing, commissioning, acceptance and quality assurance (QA) for verification of the quality of the radiation delivery in standard and conformal radiotherapy, as well as partial QA in IMRT (Intensity Modulated Radio Therapy) applications, comprising an active area consisting in individual radiation detectors being either in the form of ionisation chamber pixels or in the form of diode pixels, characterised in that the active area consists in a limited number of lines of pixels.

Fig. 1b

## Description

### Field of the Invention

The present invention is related to a dosimetry device having a configuration allowing accelerator factory testing, commissioning, acceptance and quality assurance (QA) for verification of the quality of the radiation delivery in standard and conformal radiotherapy, as well as partial QA in IMRT (intensity modulated radio therapy) applications and tomography applications.

### State of the Art

In treating patients with radiation, the radiation oncologist prescribes a treatment regimen (including the radiation dose) whose goal is to cure or control the disease while minimizing complications to normal tissues. In general, published clinical and experimental results demonstrate that the response of tumours and normal tissues to radiation is highly variable. Moreover, for some tumours and normal tissues, the dose response curves may be very steep in the therapeutic dose range, i.e., a small change in dose can result in a large change in clinical response. In addition, the prescribed radiation dose to the tumour is usually, by necessity, constrained by the tolerance dose of the surrounding normal tissues. Consequently, since the "window" for optimal treatment can be quite narrow, the radiation dose must be delivered accurately and consistently.

Delivery of treatment in an accurate and consistent manner is by no means easy to achieve, since the radiation therapy process is a complex interweaving of a number of related tasks for designing and delivering radiation treatments.

Therefore, general prescriptions including particular dosimetry tests have been established in order to have a control and an (on-line) verification of said radiation.

Usually, these tests are performed at first at the release of a new radiotherapy device from production and then at its installation in a clinical institution in order to control that the expected dose is really delivered as required. These tests are performed by the accelerator manufacturers for the release from production and after any important maintenance intervention and by the medical physicist of the clinical institution for the device acceptance and commissioning in order to prove the compliance of the equipment to the norms set by regulation authorities.

Furthermore, regular tests based on a routine schedule have also to be performed by the users in order to check the behaviour of the radiotherapy device. Again, dosimetry tests are therefore performed. They are defined as quality assurance. The prescriptions for performing such tests for quality assurance are described in details in AAPM REPORT NO. 46 "Comprehensive QA for Radiation Oncology", published for the American Association of Physicists in Medicine by the American Institute of Physics, reprinted from MEDICAL PHYSICS, Volume 21, Issue 4, 1994.

A beam profiler device is needed in order to have the required dosimetry test. One of the most known instruments used in order to perform such beam profiling is the "water phantom" instrument.

This dosimetry instrument is in the form of a water tank which uses a single detector that moves immersed in the water inside the tank, recording the dose profiles in the three dimensions. Although this device represents the golden standard in accelerator commissioning, acceptance and routine QA, thanks to its flexibility in recording the dose profiles, performing the needed measurements with it is a cumbersome and long lasting task: the water tank is an heavy and bulky device and takes long to set it up, the scans take time to be performed as only one detector is present, etc.

Other possible dosimetry equipments include devices comprising unitary radiation detectors performing such measurement, possibly in the form of matrixes or arrays. Two main families of such devices can be described, the one using diodes and the one using ionisation chambers.

An example of these devices using diodes is described in US-A-6125335 wherein a beam profiler is in the form of an array of 46 sensor diodes, S1-S46 and 4 off-axis horizontal sensor diodes, S48-S51. An example of a typical beam profiler is the off-the-shelf multi-sensor radiation detector array entitled: Profiler Model 1170, manufactured by the assignee of the subject invention, Sun Nuclear Corporation of Melbourne, Fla. The Sun Nuclear Profiler generates a real time graphic image which is a trace of individual data points spaced approximately 5 mm apart and updated each second. The 46 diodes, S1-S46 and off-axis detectors S48-S51 provide a real time profile of the emitted radiation beams and off axis analysis. However, the main problem of these devices is the fact that they use diodes which do have a non-linear response, requiring accordingly complex calibration methods such as the one described in document US-A-6125335.

Furthermore, compared to dosimetry equipments based on ionisation chambers, a device using diodes has a cost price per unit (pixel) higher than a device using ionisation chambers.

Another family consists in dosimetry instruments which implement arrays of ionisation chambers instead of diodes.

Recently, very efficient ionisation chamber arrays have been developed by Bonin et al. in "A pixel chamber to monitor the beam performances in hadron therapy" Nuclear Instruments and Methods in Physics Research A 519 (2004) - 674-686. This document describes a device made up of a 2-D array of 1024 successive ionisation chambers arranged in a regular matrix of 32 x 32 pixels. The general principle of an ionisation chamber is as follows: a high voltage is applied between two parallel electrodes. A gas (here air or nitrogen) present between the plates is ionised by the beam passing perpendicularly to the planes. As a result of the electric field, the ions are collected on the electrodes, and the charge can be measured. As the creation of one electron-ion pair requires a known average energy, depending on the gas and on the irradiation type, the collected charge is directly proportional to the energy deposited in the gas. A recycling integrator circuit provides a 16-bit counter proportional to the detected charge. The recycling integrator was developed as a 0,8 $\mu$m CMOS technology chip (TERA06) by INFN (Istituto Nazionale di Fisica Nucleare, Torino). Each of these chips provides 64 channels. The minimum detectable charge is adjustable between 50fC and 800fC, and the read rate in the linear region can be as high as 5 MHz.

As described above, the monitor comprises 32 x 32 air vented ionisation chamber pixels arranged in a matrix with a pitch of 7.5 mm.

Although this device is considered to be very efficient, its geometry, a matrix of 32 x 32 ionisation chamber pixels, is best adapted to the complexity of the dose distributions delivered in IMRT but well too complex and expensive for standard and conformal RT, where normally only the profiles on the main axis and diagonals are needed. Furthermore, the complexity of arranging such a high number of detectors and their read-outs in a matrix, had an impact on the dimensions of such a device, which will not be large enough to cover all the field dimensions which need to be checked both in routine QA and in accelerator commissioning/acceptance.

Although some of the devices of the state of the art are providing energy measurements e.g. by means of build-up plates of different thicknesses, they always need for the user to enter several times into the treatment room to perform the measurement with the required build-up plate.

Accordingly, no practical solution is proposed to perform an easy energy measurement with such a device.

## Aims of the Invention

The present invention aims to provide a dosimetry device that does not present the drawbacks of the state of the art.

In particular, the present invention aims to provide a dosimetry device which requires a limited number of individual radiation detectors while still providing the required accuracy, ease of use and fast operation.

Furthermore, the present invention aims to provide a device which will not need the user to enter several times in the treatment room to perform the measurements.

A further aim of the present invention is to provide a device which also allows the fast and efficient measurement of the beam energy of electrons and photons.

Optionally, the present invention aims to provide a device which can be used for factory testing, commissioning, acceptance and quality assurance (QA).

A further aim of this invention is to provide a limited set of tests to be used in IMRT QA.

Finally, the present invention aims to provide a device having a reasonable price.

## Summary of the Invention

A first aspect of the present invention is related to a dosimetry device for verification of the quality of the radiation delivery in standard and conformal radiation therapy and in particular in IMRT (Intensity Modulated Radio Therapy) applications, comprising an active area consisting in individual radiation detectors being in the form either of ionisation chamber (IC) pixels or diodes pixels, characterised in that the active area consists in a limited number of lines of pixels.

Preferably, the limited number is at least 2 and strictly less than the smallest value of two integers (n, m), n and m being the number of pixels in the two first lines (e.g. one column of n pixels and one row of m pixels being preferably orthogonal to each other).

According to one preferred embodiment of the present invention, the active area consists in at least one set of two lines of pixels, said two lines being essentially orthogonal to each other. in such a case, it is preferable that the detectors constituting the pixels are in the form of ionisation chamber pixels.

According to another embodiment of the present invention, the active area consists in at least two sets of two lines of pixels, said two lines of each set being essentially orthogonal to each other. Advantageously, the first set of lines is rotated over an angle compared to the second set of lines, which preferably corresponds essentially to 45 degrees.

Advantageously, some of said lines of pixels are passing through the centre of said active area.

The majority of pixels of the active area can be located on said lines. Said lines have at least 3 pixels, but preferably at least 10 pixels and more preferably at least 20 pixels.

Another aspect of the present invention provides means for determining the field penumbra, the field width and any beam characteristics defined in the field penumbra region or in field gradients through interpolation by applying the Fermi function.

According to a preferred embodiment of the present invention, the active area further comprises extra pixels dedicated to the energy measurement of electrons or photons, the extra pixels being linked to energy absorbers being in the form of material degraders. Said material degraders for the electron or the photon energy measurement can either be in the form of bumps of different thicknesses and/or in the form of bumps or recesses with inserts of different materials. According to the preferred embodiment, the material degraders for the electron measurement can essentially be positioned in each quarter or octant defined by one set or two sets of lines dividing the active area. The degraders do not prevent the use of the same build-up plate for the pixels located on the lines.

According to a second aspect, the present invention is related to a dosimetry device comprising an active area consisting in individual radiation detectors being in the form either of ionisation chamber (IC) pixels or diodes pixels, wherein the active area comprises extra pixels dedicated to the measurement and determination of the projection of the leaf width at the isocenter, said extra pixels being comprised in two lateral bands, which are positioned parallel to the lateral side of the dosimetry device, and preferably at the lateral extremity of said device. Optionally said active area can further comprise an extra band of detectors being positioned on the central axis between the two lateral bands.

According to a first embodiment, each band consists in a few (3 to 6) lines (columns) of pixels. Of course, the several lateral pixels belonging to the several columns should also be located on a same line (row).

According to a second embodiment, each band consists in a single column of individual detectors.

The means for the determination of the projection of the leaf comprises means for determining the field penumbra, the field width and any beam characteristics defined in the field penumbra region or in field gradients through interpolation by applying the Fermi function.

Preferably, and according to all the several aspects of the present invention, said pixels are ionisation chamber pixels and are processed through IC technology.

In such a case, the dosimetry device essentially consists in a stack of three main planar components:

- a top layer, constituting the electrode top layer;
- a mid layer wherein drilled holes constitute the IC pixels defining accordingly the active area;
- a bottom layer constituting the segmented electrode and carrying also the electronic chips.

Nowadays, the maximum size of the surface of the active area for a dosimetry device having ionisation chamber pixels is a surface of a few $cm^2$, e.g. a square of $0.5 \times 0.5\,m^2$. Preferably, each detector has a cylindrical shape with a diameter equal or higher than 3 mm and a pitch equal or higher than 5 mm. Another possibility is to have a square shape for the section of each detector. This is particularly advantageous in the case of the detectors being positioned in the lateral bands assuming the measurement and the determination of the leaf position of the beam.

The main advantage for having such IC pixels in the dosimetry device is that said device does not need any complex calibration method because the measurement elements constituting the ionisation chamber shows a linear response to the dose delivered. Furthermore, the cost price is lower for IC devices than for diode devices.

Another possibility, and this is according to all the several aspects of the present invention, said pixels are diodes, which do have some drawbacks such as they need a calibration and show a drift under radiation.

However, they also show some advantages, i.e. their dimensions are smaller than the ones for ionisation chamber pixels. According, they can be packed in a much closer matter then the IC pixels, and therefore they give a good resolution at a reasonable cost.

Another aspect of the present invention concerns the use of the dosimetry device according to the invention to perform the measurement of field characteristics with field sizes bigger than the detector active area, by positioning said dosimetry device on a frame or holder which is moving within the beam field on a source - axis distance less than or equal to 100 cm.

## Brief description of the drawings

Figs. 1a and 1b represent an exploded view of a device and a top view of the mid-layer of said device according to one preferred embodiment of the present invention.

Figs. 2a and 2b represent an exploded view of a device and a top view of the mid-layer of said device according to another preferred embodiment of the present invention.

Fig. 3 is representing an example of simulated edge of field profile, as measured with one row of pixels and as fitted with a Fermi function.

Fig. 4 represents the measurement points for electron energy measurement obtained by eight pixel ionisation chambers beneath the corresponding energy absorbers present on the electron build-up plate of the device as described in Fig. 1.

Fig. 5 represents an example of a measurement of the photon energy performed by means of two pixel ionisation chambers beneath the corresponding two energy absorbers present on the photon build-up plate of the device as described in Fig. 1.

## Detailed description of preferred embodiments of the present invention

In relation to the appended drawings, the present invention is described in details for an embodiment using ionisation chamber technology.

It is apparent however that a person skilled in the art can imagine several other equivalent embodiments or other ways of executing the present invention, such as suggesting diodes instead of ionisation chambers for the pixels, the spirit and the scope of the present invention being limited only by the terms of the claims.

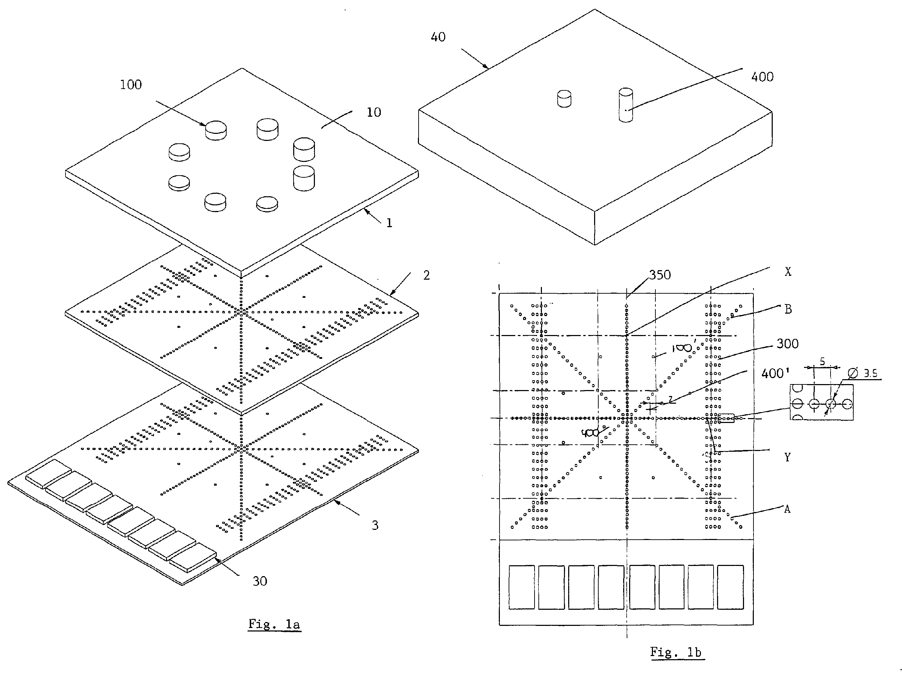

Fig. 1a represents an exploded view of a device according to a preferred embodiment of the present invention which is using ionisation chambers in order to perform dosimetry tests. This dosimetry device essentially consists in a stack of three main planar components:

1) a top layer (1), constituting the electrode top layer;
2) a mid layer (2) wherein drilled holes (20) constitute the ionisation chambers defining accordingly the active area;
3) a bottom layer (3) being the segmented electrode and carrying also the electronic chips and the tracks bringing the signals from the ionisation chambers to the electronic chips(30); said layers being covered by a build-up plate (10 or 40) with energy absorbers (100' or 400').

The general principle of said ionisation chambers is again explained hereunder.

The radiation traversing the chambers ionises the gas present in between the top layer and the bottom layer. When a high voltage is applied between the two electrodes, an electric field is created and the ions are collected on the electrodes thereby creating a signal which can be measured.

The technology used for creating these ionisation chambers can be any one described in the state of the art and can be for example the pixel chambers developed by BONIN et al. using the so-called TERA electronics.

According to a preferred embodiment, the dosimetry device can comprise, in particular, the following layers:

- as the top layer constituting the top electrode: a 50$\mu$m thick printed Polyimide (Pyralux AP 8525R) layer covered with 25$\mu$m carbon layers on both sides. The inner layer is structured according to the holes of the mid layer with a round shape of e.g. 0.1mm smaller than the holes diameter (diam. 3.5mm). The top carbon layer acts as an EMC shield;
- as the mid layer: a layer which consists in an approximately 5mm thick pure polycarbonate plate with drilled holes of e.g. 3.5mm diameter and spaced from each other over a length of more than 5mm; ventilation can also be provided; therefore the mid layer is laminated to the top and to the segmented electrode on the bottom layer by means of adhesive dots that act as standoffs;
- as the bottom layer: a layer manufactured according to printed circuit board (PCB) techniques. An example of such a bottom layer using PCB techniques is described in the US provisional application filed in the name of the inventors of the present patent application on May 27, 2005, this document being incorporated by reference in the present application.

Fig. 1b represents a preferred configuration of an active area for one preferred embodiment of the present invention, wherein the active area is constituted by individual radiation detectors being either ionisation chambers (IC) pixels. However, exactly the same configuration can apply to diode pixels.

According to the present invention, said configuration consists in at least one set of two essentially orthogonal lines of pixels. Said lines are preferably passing through the centre of the active area and comprise preferably the majority of the pixels present in said active area. In such case, one should assume that the two lines comprise m and n pixels respectively, n and m being integers and higher than 1.

More preferably, said preferred configuration consists in at least two sets of essentially orthogonal lines of pixels, said lines passing preferably through the centre of the active area and comprising preferably the majority of the pixels present in said active area.

One can say that the configuration of the active area consists in a limited number of lines. By "limited", it should be understood at least 2 and strictly less than the smallest value of two integers (n, m), n and m being the number of pixels comprised by the two lines respectively. In order to have a line, n and m should be an integer of at least 3 but preferably of at least 10, and more preferably of at least 20.

Furthermore, "limited" means equal to or less than 8, more preferably equal to or less than 4, more preferably equal to 2.

However, said configuration of pixels does not cover all the surface of the mid layer. Said configuration cannot be considered as a matrix defined as a n x m pixels matrix which corresponds to an embodiment of the state of the art.

Accordingly, the total number of pixels of the present invention is considered to be a linear function f (n, m) or f(n) if n = m, while the number of pixels for an embodiment of the state of the art (matrix configuration) is a function f(n x m) or f ($n^2$) if n = m.

Another way to understand the present invention can be as follows: only very few pixels are present in the area divided by the lines, namely in the quarters if the division of the active area is performed by one set of two lines, or in the octants if the active area is divided by four lines.

If we compare the number of pixels used in a device built according to the state of the art and in a device according to the present invention, it is obvious that the present invention suggests a device with a lower number of pixels and, accordingly, with a lower price. But if we build a device having a larger number of pixels or even the same number of pixels as, for instance, the device as described by Bonin et al., namely a dosimetry device having 32 x 32 pixels (1024 pixels), a device according to the preferred embodiment of the invention having two sets of two lines of 256 pixels each will show either a surface for the active area (up to 8x, which is actually unrealistic) higher than the device suggested by Bonin et al. with comparable results relating to the measurements of the beam profiles or with a configuration with a (down to 8x, which is actually unrealistic) smaller pitch than the device suggested by Bonin et al. and then able to give a more efficient result for the measurement.

Of course, in practice, a compromise of these several requirements will be obtained:

- reduction of the total number of pixels,
- while increasing the surface of the active area,
- while decreasing the pitch between two consecutive pixels.

As represented on Fig. 1b, the two rows (X and Y) of the first set essentially consist in the median lines (longitudinal and transversal axes) of the mid layer, while the two rows (A and B) of the second set essentially consist in the diagonals of said mid layer, all of them passing through the centre O, the shape of the active area of said mid layer being essentially a square of e.g. 200 x 200 $mm^2$ to 400 x 400 $mm^2$.

Preferably, each row or line (X, Y, A or B) comprises at least 50 pixels with a pitch less than 7 mm and preferably less than 5 mm and even preferably less than 4 mm.

According to a particular embodiment, the pitch on the median lines and the diagonal lines can be the same.

According to another preferred embodiment, the pitches are different on the median lines and on the diagonal lines. For instance, the pitch on the diagonal lines corresponds to $\sqrt{2}$ times the pitch on the median lines.

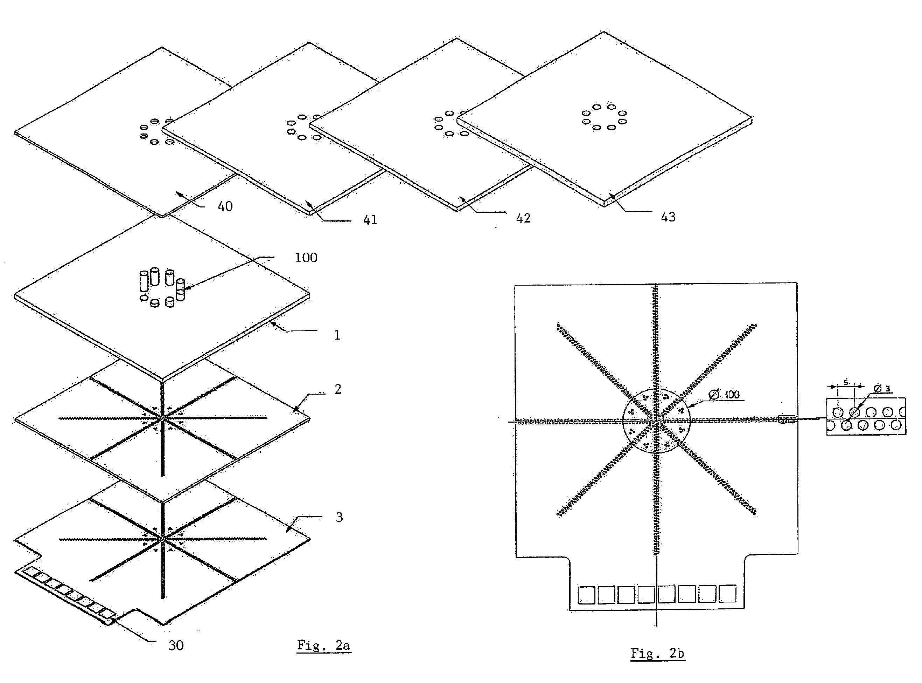

By "line", "row" or "column", it should be understood either individual pixels placed successively (one after the other) or few pixels placed within a certain width (as small as possible) for said row or line such as represented in Figs. 2a and 2b, said "line", "row" or "column" comprises at least 3 pixels, preferably at least 10 pixels and more preferably at least 20 pixels.

The spatial resolution of the measurements at the field penumbra can be, according to a preferred embodiment, improved in respect to the spacing of the pixels by applying a data interpolation based on the well known Fermi function:

$$F(x) = \frac{1}{1 + e^{a(x_0 - x)}}$$

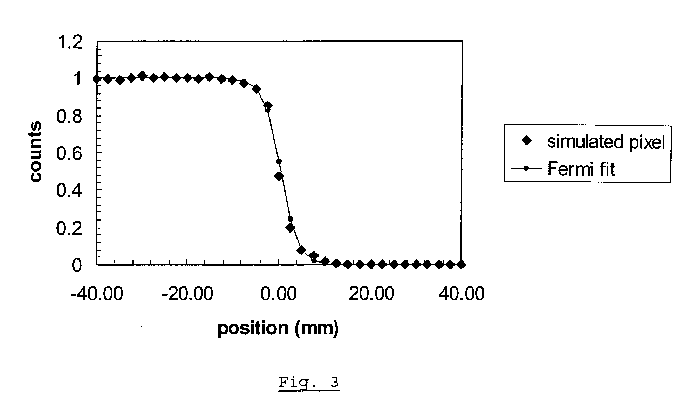

where a represents the penumbra p through the relation $a=2.4/p$, $x_0$ represents the 50% position in the penumbra and the dose in the homogeneous part of the field is normalized to 1, as shown in Fig. 3. By applying such an interpolation, the precision in identifying the field penumbra, the field width and all the field related quantities determined in the penumbra region is improved to a fraction of 1 mm for a pitch of 5 mm.

The fact that the individual radiation detectors are placed in rows will give the sufficient information in order to calculate and design the beam profiles in a one-step measurement, thereby avoiding any moving or manipulation of the dosimetry device. Said measurement can be considered as instantaneous. Thanks to its fast electronics, the device can also be used as a "real time" measurement device, allowing the steering of some accelerator parameters while observing the modifications induced on the measured profiles.

Furthermore, the fact that the pixels present in lines are not covering the whole surface of the mid layer will obviously reduce the price of the electronics attached to said dosimetry device and accordingly the final price of the dosimetry device.

According to another aspect of the present invention, in order to be able to cover field sizes bigger than the detector active area, the device can be coupled with a frame holding it at a variable source - axis distance (SAD) less or equal to 100 cm.

Optionally, to provide a measurement dedicated to IMRT QA and more specifically to Multi Leaf (ML) Collimator QA as represented in Figs. 1a and 1b, two lateral (vertical) bands 300 of pixels are available at a distance of e.g. 5 to 10 cm from the central axis. Each band is composed of a few (3 to 6) columns or rows of pixels present at the lateral sides (edges) of the mid layer with a spacing of 0.5 cm corresponding to the leaf size at the isocenter plane of measurement, in the longitudinal (vertical) direction with a pitch of 4 to 8 mm in the transversal (horizontal) direction. Optionally, a central band 350 can be provided with also a few pixels. When the several MLs are positioned as to give a 20 x 20 $cm^2$ field on the detector, each transversal row of a few pixels is then providing the measurement of the position of a single ML, again by using a Fermian fit on the measured penumbra, with a spatial precision of a fraction of 1 mm.

Another embodiment can provide only one row of pixels on each lateral side of the dosimetry device which are dedicated to the leaf measurement.

According to another important aspect of the present invention, extra pixels 100 can be dedicated to the measurement of the energy of the electrons. Therefore, on the cover (top plate) 10 covering the top layer of the dosimetry device, and dedicated to the electron measurements, a set of degraders 100' of different materials having a different water-equivalent thickness from one degrader to the other are embedded in the cover. Preferably, the combination of materials and thicknesses of said degraders gives an increasing water-equivalent thickness within a range comprised between 5 to 90 mm. Said cover has a water-equivalent thickness of at least 10 mm. Of course, said degraders 100' are in direct connection with the underlining pixels 100 which will be able to perform the required energy measurement.

According to the preferred embodiment described in Fig. 1, eight degraders and corresponding pixels are placed on the periphery of a circle included in a 20 x 20 $cm^2$ field. Of course, said extra pixels should not be placed in a too close vicinity to the pixels present in the several rows intended to measure the beam profile.

According to another important feature of the present invention, extra pixels 400 can be dedicated to the measurement of the energy of the photons. Therefore, on a build-up plate or cover 40 covering the top layer of the dosimetry device and dedicated to the photon measurements, two extra energy absorbers 400' are present within a 10 x 10 cm² field at isocenter. Said degraders have a thickness corresponding to 10 and 20 cm water-equivalent respectively, while the cover has a thickness of 50 mm water-equivalent. Again, said degraders are in direct connection with the underlining pixels 400 which will be able to perform the required energy measurement.

Preferably, the detectors dedicated to the measurement of energy show dimensions slightly higher than the detectors present on the rows, (X, Y, A and B).

Two possibilities are provided in order to realise the variation of the water-equivalence thickness and accordingly the variation of the energy absorption:

- either having bumps with different thicknesses,
- or having bumps on the cover or recesses with inserts in the thickness of the cover, made of different materials.

Once again, the fact that several energy absorbers are present on each cover will allow an energy measurement which can be performed in one step without any moving or any manipulation of said dosimetry device.

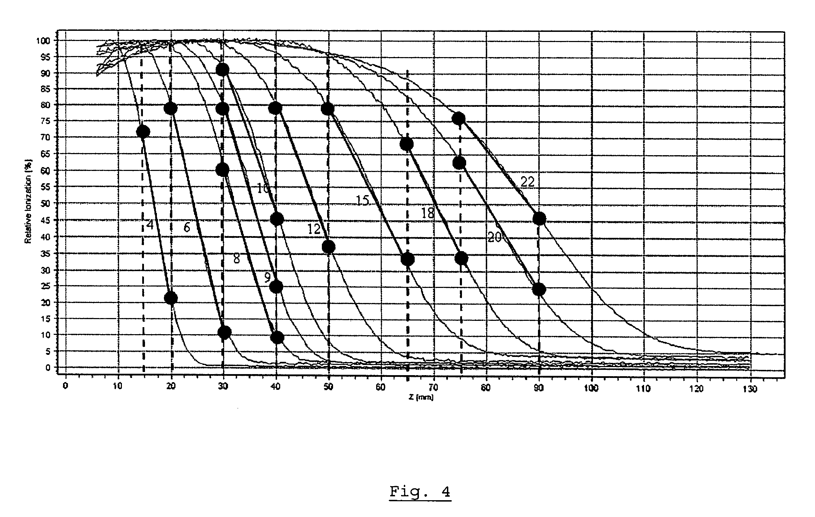

The results of the energy measurement performed with eight energy absorbers will permit to uniquely identify each beam energy by determining the slope or the R50 value of each curve, from two measurement points on the slope of each electron energy measurement as described in Fig. 4.

Fig. 4 is representing a particular example of a set of electron DPPs corresponding to energies of 4, 6, 8, 9, 10, 12, 15, 18, 20, 22 MeV, with corresponding set of water-equivalent depths identifying the steepness of the slope or the R50 value of each DPP curve.

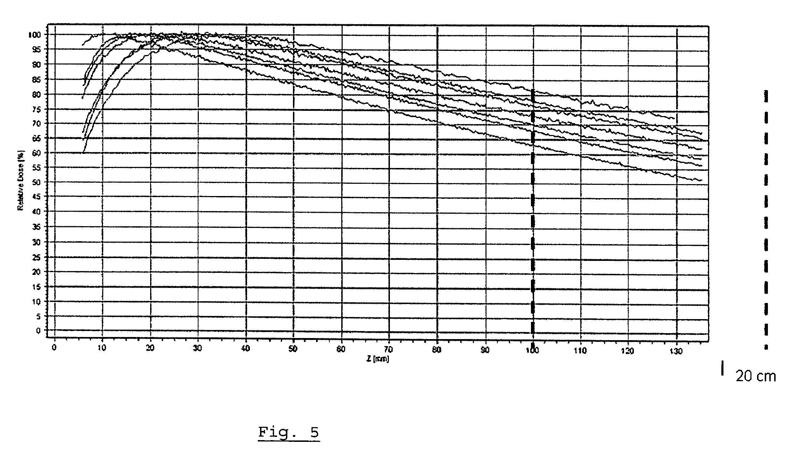

The results of the energy measurement performed with 2 photon energy absorbers will permit to determine the TPR 20.10 for each photon energy measurement as represented in Fig. 5.

Fig. 5 is representing a particular example of a set of photon DPPs corresponding to energies of 4, 6, 8, 9, 10, 12, 15, 18, 20, 22 MeV, with corresponding 2 water-equivalent depths identifying each DDP curve by its TPR 20,10 ratio.

**Claims**

1. Dosimetry device for verification of the quality of the radiation delivery in standard and conformal radiation therapy and in particular for IMRT (Intensity Modulated Radio Therapy) applications, comprising an active area consisting in individual radiation detectors being either in the form of ionisation chamber (IC) pixels or in the form of diode pixels, **characterised in that** the active area consists in a limited number of lines of pixels.

2. Dosimetry device according to claim 1, **characterised in that** the limited number is at least 2 and strictly less than the smallest value of two integers (n, m), n and m being the number of pixels in the first two lines.

3. Dosimetry device according to claim 2, **characterised in that** the active area consists in at least one set of two lines (A and B or X and Y) of pixels, said two lines being essentially orthogonal to each other.

4. Dosimetry device according to claim 3, **characterised in that** the active area consists in at least two sets of two lines of pixels, said two lines of each set being essentially orthogonal to each other.

5. Dosimetry device according to claim 4, **characterised in that** the first set of lines (X, Y) is rotated over an angle compared to the second set of lines (A, B).

6. Dosimetry device according to claim 5, **characterised in that** the angle corresponds essentially to 45 degrees.

7. Dosimetry device according to any one of the preceding claims, **characterised in that** some of said lines of pixels are passing through the centre of said active area.

8. Dosimetry device according to any one of the preceding claims, **characterised in that** the majority of pixels of the active area is located on said lines (A and B and/or X and Y).

9. Dosimetry device according to any of the preceding claims, **characterised in that** the active area further comprises pixels (100, 400) dedicated to the energy measurement of electrons or photons, said extra pixels (100 or 400) being linked to energy absorbers.

10. Dosimetry device according to claim 9, **characterised in that** the material degraders (100', 400') constituting the energy absorbers for the electron or the photon energy measurement are either in the form of bumps of different materials and/or different thicknesses and/or in the form of bumps or recesses with inserts of different materials and/or different thicknesses.

11. Dosimetry device according to claim 10, **characterised in that** the material degraders (100) for the electron measurement are essentially positioned in each quarter or octant defined by one set or two sets of lines (A and B and/or X and Y) dividing the active area.

12. Dosimetry device for verification of the quality of the radiation delivery in standard and conformal radiation therapy and in particular for IMRT (Intensity Modulated Radio Therapy) applications, comprising an active area consisting in individual radiation detectors being either in the form of ionisation chamber (IC) pixels or in the form of diode pixels, **characterised in that** the active area comprises pixels (300) dedicated to the measurement and determination of the projection of the leaf width at the isocenter of the beam, said pixels being positioned on lateral bands being located at the lateral extremity of the device.

13. Dosimetry device according to claim 12, **characterised in that** it comprises means for determining the field penumbra, the field width and any beam characteristics defined in the field penumbra region or in field gradients through interpolation by applying the Fermi function.

14. Dosimetry device according to claim 12 or 13, **characterised in that** the pixels (300) dedicated to the measurement and determination of the projection of the leaf width are positioned in the form of lateral bands of at least three parallel columns of pixels.

15. Dosimetry device according to any one of the preceding claims 12 to 14, **characterised in that** the active area further comprises an extra band of detectors being positioned in the central axis between the two lateral bands.

16. Dosimetry device according to claim 12 or 13, **characterised in that** each band consists in a single column of radiation detectors.

17. Use of the dosimetry device according to any one of the preceding claims in order to perform the measurement of field characteristics with field sizes bigger than the detector active area, by positioning said dosimetry device on a frame or holder within the beam field on a source - surface of detector distance (SSD) less than or equal to 100 cm.

Fig. 1b

Fig. 1a

Fig. 2b

Fig. 2a

Fig. 3

Fig. 4

Fig. 5

European Patent
Office

**EUROPEAN SEARCH REPORT**

Application Number

EP 05 44 7206

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BONIN R ET AL: "A pixel chamber to monitor the beam performances in hadron therapy" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, vol. 519, no. 3, 1 March 2004 (2004-03-01), pages 674-686, XP004492326 ISSN: 0168-9002 * the whole document * | 1-8, 12-17 | A61N5/10 G01T1/29 |
| X | US 6 125 335 A (SIMON ET AL) 26 September 2000 (2000-09-26) * the whole document * | 1-8, 12-17 | |
| X | AMERIO S ET AL: "Dosimetric characterization of a large area pixel-segmented ionization chamber" MEDICAL PHYSICS, AMERICAN INSTITUTE OF PHYSICS. NEW YORK, US, vol. 31, no. 2, February 2004 (2004-02), pages 414-420, XP012074787 ISSN: 0094-2405 * the whole document * | 1-8, 12-17 | **TECHNICAL FIELDS SEARCHED (IPC)** A61N G01T |
| A | DE 101 43 609 A1 (SCHUSTER MEDIZINISCHE SYSTEME GMBH) 27 March 2003 (2003-03-27) * paragraph [0014]; figures * | 3-8 | |

-/--

The present search report has been drawn up for all claims

| Place of search | Date of completion of the search | Examiner |
|---|---|---|
| Munich | 3 February 2006 | Rodríguez Cossío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**EUROPEAN SEARCH REPORT**

Application Number

EP 05 44 7206

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BRUSASCO C ET AL: "A dosimetry system for fast measurement of 3D depth-dose profiles in charged-particle tumor therapy with scanning techniques" NUCLEAR INSTRUMENTS & METHODS IN PHYSICS RESEARCH, SECTION - B: BEAM INTERACTIONS WITH MATERIALS AND ATOMS, ELSEVIER, AMSTERDAM, NL, vol. 168, no. 4, August 2000 (2000-08), pages 578-592, XP004206343 ISSN: 0168-583X ----- | | |
| A | DE 38 44 716 C2 (MITSUBISHI DENKI K.K., TOKIO/TOKYO) 22 February 2001 (2001-02-22) ----- | | |

TECHNICAL FIELDS SEARCHED (IPC)

The present search report has been drawn up for all claims

| Place of search | Date of completion of the search | Examiner |
|---|---|---|
| Munich | 3 February 2006 | Rodríguez Cossío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P04C01)

**EP 1 747 799 A1**

## ANNEX TO THE EUROPEAN SEARCH REPORT
## ON EUROPEAN PATENT APPLICATION NO.

EP 05 44 7206

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2006

| Patent document cited in search report | | Publication date | Patent family member(s) | Publication date |
|---|---|---|---|---|
| US 6125335 | A | 26-09-2000 | NONE | |
| DE 10143609 | A1 | 27-03-2003 | NONE | |
| DE 3844716 | C2 | 22-02-2001 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6125335 A

**Non-patent literature cited in the description**

- Comprehensive QA for Radiation Oncology. American Association of Physicists in Medicine by the American Institute of Physics, 1994, vol. 21

- **BONIN et al.** A pixel chamber to monitor the beam performances in hadron therapy. *Nuclear Instruments and Methods in Physics Research A,* 2004, vol. 519, 674-686